# EUROPEAN PATENT APPLICATION

(11) **EP 4 066 885 A1**
(43) Date of publication of application: **05.10.2022**
(21) Application number: 22165120.1
(22) Date of filing: 29.03.2022
(51) Int. Cl.: A61N 1/375, A61B 5/00, A61N 1/378

(54) **IMPLANTABLE MEDICAL DEVICE AND METHOD OF FORMING SAME**

(30) Priority: 31.03.2021 US 202163168556 P; 15.03.2022 US 202217694990
(71) Applicant: Medtronic, Inc., Minneapolis, Minnesota 55432 (US)
(72) Inventor: Iyer, Rajesh V., Minneapolis, 55432 (US); Young, Paul B., Minneapolis, 55432 (US); Thom, Andrew J., Minneapolis, 55432 (US); Roles, Randy S., Minneapolis, 55432 (US)
(74) Representative: Zimmermann & Partner Patentanwälte mbB

(57) **Abstract**

Various embodiments of an implantable medical device and a wireless energy transfer system that includes the implantable medical device are disclosed. The device includes a housing that has a first major surface and a second major surface, a sidewall that extends between the first major surface and the second major surface, and an opening disposed in the sidewall. The device further includes a window disposed on at least one of the first major surface or second major surface of the housing, and a nonconductive material disposed on the housing, wherein the opening is hermetically sealed by the nonconductive material. At least one of the window or the sealed opening is adapted to transmit electromagnetic energy.

## Description

### CROSS-REFERENCE TO RELATED APPLICATION

This application claims the benefit of U.S. Provisional Application No. 63/168,556, filed March 31, 2021, the disclosure of which is incorporated by reference herein in its entirety.

### TECHNICAL FIELD

This disclosure generally relates to an implantable medical device and in particular a wireless energy transfer system that includes the implantable medical device.

### BACKGROUND

Wireless energy transfer systems such as transcutaneous energy transfer (TET) systems are used to supply power to implantable medical devices that are implanted within a human body. An electromagnetic field generated by a transmitting coil outside the body can transmit power across a cutaneous (skin) barrier to a magnetic receiving or charging coil implanted within the body. The receiving coil can then transfer the received power to one or more implantable devices and to one or more power sources (e.g., batteries) implanted within the body to charge the power source. Such systems efficiently generate and wirelessly transmit a sufficient amount of energy to power one or more implanted devices while maintaining the system's efficiency and overall convenience of user.

### SUMMARY

The techniques of this disclosure generally relate to an implantable medical device and a wireless energy transfer system that includes the implantable medical device. The device can include one or more openings that are disposed in a sidewall of a housing. The openings can be hermetically sealed by a nonconductive material disposed on the housing. The device can also include one or more windows disposed on at least one of a first major surface or a second major surface of the housing. At least one of the window or the sealed openings can be adapted to transmit electromagnetic energy such that one or more electronic components disposed within the housing of the device can receive energy from an external charging component that is adapted to direct electromagnetic energy to the device.

This disclosure includes without limitation the following clauses:
Clause 1: An implantable medical device that includes a housing that has a first major surface and a second major surface, a sidewall that extends between the first major surface and the second major surface, and an opening disposed in the sidewall. The device further includes a window disposed on at least one of the first major surface or the second major surface of the housing, and a nonconductive material disposed on the housing, where the opening is hermetically sealed by the nonconductive material. At least one of the window or the sealed opening is adapted to transmit electromagnetic energy.
Clause 2: The device of Clause 1, further including a charging coil disposed within the housing and adapted to receive electromagnetic energy directed through the window or the sealed opening.
Clause 3: The device of Clause 2, further including an electronic component disposed within the housing and electrically connected to the charging coil.
Clause 4: The device of Clause 3, where the electronic component includes a battery.
Clause 5: The device of any one of Clauses 1-4, where the window includes a nonconductive material.
Clause 6: The device of Clause 5, where the window includes at least one of sapphire, glass, zirconia, or alumina.
Clause 7: The device of any one of Clauses 1-6, where the housing includes titanium.
Clause 8: The device of any one of Clauses 1-7, where the window is disposed on the first major surface of the housing, and further where the housing includes a second window disposed on the second major surface of the housing.
Clause 9: The device of any one of Clauses 1-8, where the housing includes a plurality of openings disposed in the sidewall, where each opening is hermetically sealed by the nonconductive material.
Clause 10: The device of any one of Clauses 1-9, where the nonconductive material includes at least one of sapphire, glass, zirconia, or alumina.
Clause 11: The device of any one of Clauses 1-10, where the window is hermetically sealed to the housing.
Clause 12: The device of Clause 11, where the window is diffusion bonded to the housing.
Clause 13: The device of Clause 12, where the window is laser-assisted diffusion bonded to the housing such that a bond line is formed between the window and the housing.
Clause 14: The device of Clause 11, where the window is brazed to the housing.
Clause 15: A pacemaker including the implantable medical device of any one of Clauses 1-14.
Clause 16: A leadless cardiac monitor including the implantable medical device of any one of Clauses 1-14.
Clause 17: A wireless energy transfer system that includes an external charging component adapted to emit electromagnetic energy, and an implantable medical device. The device includes a housing that has a first major surface and a second major surface, a sidewall that extends between the first major surface and the second major surface, and an opening disposed in the sidewall. The device further includes a window disposed on at least one of the first major surface or second major surface of the housing; nonconductive material disposed on the housing, where the opening is hermetically sealed by the nonconductive material; and a charging coil disposed within the housing and adapted to receive the electromagnetic energy emitted by the external charging component. The device also includes an electronic component disposed within the housing and electrically connected to the charging coil. At least one of the window or the sealed opening is adapted to transmit electromagnetic energy to the charging coil disposed within the housing.
Clause 18: The system of Clause 17, where the window includes a nonconductive material.
Clause 19: The system of any one of Clauses 17-18, where the housing of the implantable medical device includes titanium.
Clause 20: The system of any one of Clauses 17-19, where the window of the implantable medical device is disposed in the first major surface of the housing of the device, and further wherein the housing includes a second window disposed in the second major surface of the housing.
Clause 21: The system of any one of Clauses 17-20, wherein the housing of the implantable medical device includes a plurality of openings disposed in the sidewall, where each opening is hermetically sealed by the nonconductive material.
Clause 22: The system of any one of Clauses 17-21, where the window of the implantable medical device includes at least one of sapphire, glass, zirconia, or alumina.
Clause 23: The system of any one of Clauses 17-22, where the nonconductive material includes at least one of sapphire, glass, zirconia, or alumina.
Clause 24: The system of any one of Clauses 17-23, where the window is hermetically sealed to the housing.
Clause 25: The system of Clause 24, where the window is diffusion bonded to the housing.
Clause 26: The system of Clause 25, where the window is laser-assisted diffusion bonded to the housing such that a bond line is formed between the window and the housing.
Clause 27: The system of Clause 24, where the window is brazed to the housing.
Clause 28: The system of any one of Clauses 17-27, where the electronic component includes a battery.
Clause 29: The system of any one of Clauses 17-28, where the implantable medical device includes a pacemaker.
Clause 30: The system of any one of Clauses 17-28, where the implantable medical device includes a leadless cardiac monitor.
Clause 31: The system of any one of Clauses 17-30, where the implantable medical device includes a second charging coil, where the second charging coil is disposed on the nonconductive material disposed in the opening.
Clause 32: A method that includes disposing an opening in a sidewall of a housing of an implantable medical device, where the sidewall extends between a first major surface and a second major surface of the housing. The method further includes hermetically sealing the opening with a nonconductive material, and disposing a window on at least one of the first major surface or second major surface of the housing. At least one of the window or the opening is adapted to transmit electromagnetic energy.
Clause 33: The method of Clause 32, further including disposing a charging coil within the housing.
Clause 34: The method of Clause 33, further including disposing an electronic component within the housing, where the electronic component is electrically connected to the charging coil.
Clause 35: The method of any one of Clauses 33-34, further including directing electromagnetic energy through at least one of the window or opening of the housing to the charging coil.
Clause 36: The method of any one of Clauses 32-35, further including hermetically sealing the window to the housing.
Clause 37: The method of Clause 36, where hermetically sealing the window includes diffusing bonding the window to the housing.
Clause 38: The method of Clause 37, where diffusing bonding the window includes laser-assisted diffusion bonding the window to the housing.
Clause 39: The method of Clause 36, where hermetically sealing the window includes brazing the window to the housing.
Clause 40: The method of any one of Clauses 32-39, where the window is disposed in the first major surface of the housing, where the method further includes disposing a window in the second major surface of the housing.
Clause 41: The method of any one of Clauses 32-40, where disposing the opening in the sidewall includes disposing a plurality of openings in the sidewall of the housing, where hermetically sealing the opening includes hermetically sealing each opening with the nonconductive material.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a schematic top perspective view of one embodiment of an implantable medical device.
FIG. 2 is a schematic side plan view of the implantable medical device of FIG. 1.
FIG. 3 is a schematic top plan view of the implantable medical device of FIG. 1.
FIG. 4 is a schematic bottom plan view of the implantable medical device of FIG. 1.
FIG. 5 is a schematic top cross-section view of the implantable medical device of FIG. 1.
FIG. 6 is a schematic side cross-section view of a portion of the implantable medical device of FIG. 1.
FIG. 7 is a flow chart of one embodiment of a method of forming the implantable medical device of FIG. 1.
FIG. 8 is a schematic plan view of external components of a wireless energy transfer system.
FIG. 9 is a schematic plan view of internal components of the wireless energy transfer system of FIG. 8 that includes the implantable medical device of FIG. 1.
FIG. 10 is a schematic perspective view of another embodiment of an implantable medical device.

### DETAILED DESCRIPTION

The techniques of this disclosure generally relate to an implantable medical device and a wireless energy transfer system that includes the implantable medical device. The device can include one or more openings that are disposed in a sidewall of a housing. The openings can be hermetically sealed by a nonconductive material disposed on the housing. The device can also include one or more windows disposed on at least one of a first major surface or a second major surface of the housing. At least one of the window or the sealed openings can be adapted to transmit electromagnetic energy such that one or more electronic components disposed within the housing of the device can receive energy from an external charging component that is adapted to direct electromagnetic energy to the device.

Implantable medical devices increasingly require the ability to be recharged after implantation into a body of a patient. Such devices often include housings that utilize biocompatible materials such as titanium that provide a hermetically-sealed enclosure. For a charging coil or one or more electronic components disposed within the housing to receive electromagnetic energy from an external charging component, one or more portions of the housing can include a material or materials that is transmissive to such energy. Eddy currents can, however, be formed by the electromagnetic energy in one or more non-transmissive portions of the housing. For example, large eddy currents can be generated in a titanium housing by electromagnetic energy having an operating frequency of a few hundred kilohertz to several megahertz. Such large eddy currents can produce a magnetic field that interferes with a magnetic field produced by the electromagnetic energy that induced the eddy currents, thereby reducing the amount of energy received by the charging coil or other electronic components disposed within the housing.

One or more embodiments of implantable medical devices described herein can include a housing that includes one or more openings disposed therein. The device can also include one or more windows disposed in the housing. The openings and windows can form one or more high resistance tortuous paths for eddy current loops. These current loops can reduce the intensity of any interfering magnetic fields that are induced in the housing by the electromagnetic energy directed to the housing by reducing the overall surface area of the conductive materials, thereby improving recharge efficiency. Such openings can be covered or filled using any suitable nonconductive material so that the openings are hermetically sealed.

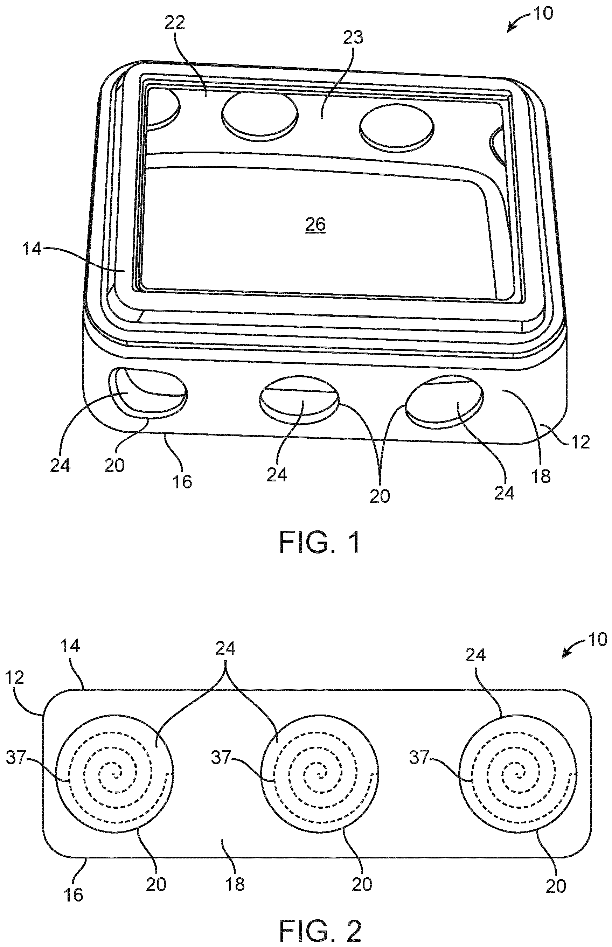

FIGS. 1-6 are various views of one embodiment of an implantable medical device 10. The device 10 includes a housing 12 having a first major surface 14 and a second major surface 16, and a sidewall 18 that extends between the first major surface and the second major surface. The housing 12 also includes one or more openings 20 disposed in the sidewall 18. The device 10 further includes a window 22 disposed in at least one of the first major surface 14 or second major surface 16 of the housing 12. The housing 12 also includes a nonconductive material 24 disposed on the housing, where one or more of the openings 20 are hermetically sealed by the nonconductive material. At least one of the window 22 or the sealed openings 20 is adapted to transmit electromagnetic energy.

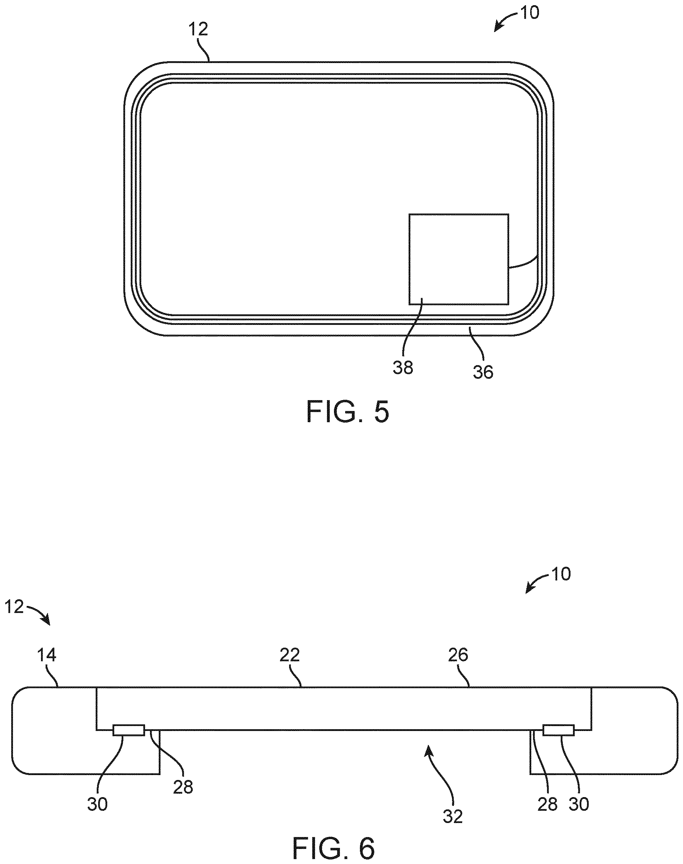

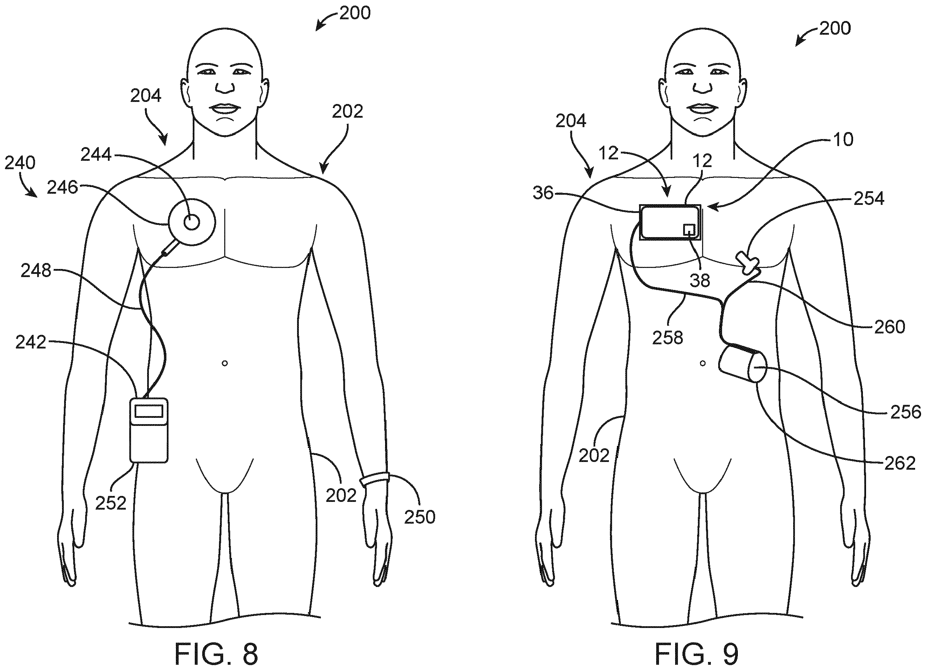

The device 10 can be any suitable device that is adapted to be implanted within a body of a patient. In one or more embodiments, the device 10 can be a pacemaker. Further, in one or more embodiments, the device 10 can be a leadless cardiac monitor. The device 10 can include any other suitable medical devices such as a defibrillator, LVAD, neurostimulator, drug pump, etc. In one or more embodiments, the device 10 can include one or more leads that are connected to one or more electronic components (e.g., electronic component 38 of FIG. 5) that are disposed within the housing 12. Further, the disclosed embodiments of implantable medical devices can be utilized with any suitable system or systems. For example, one or more embodiments of implantable medical devices can be utilized with a wireless energy transfer system, e.g., wireless energy transfer system 200 of FIGS. 8-9.

The housing 12 of the device 10 can take any suitable shape or shapes and have any suitable dimensions. Further, the housing 12 can include any suitable material or materials, e.g., at least one of titanium (e.g., any suitable grade such as grade 5 titanium), steel, niobium, tantalum, polymer, ceramic, or glass. The housing 12 can be a unitary housing. In one or more embodiments, the housing 12 can include two or more portions that are connected together using any suitable technique or techniques, e.g., welding, mechanically fastening, adhering, bonding, diffusion bonding, laser-assisted diffusion bonding, glassing, brazing, soldering, etc.

The housing 12 includes the first major surface 14 and the second major surface 16. Although depicted as including two major surfaces, the housing 12 can include any suitable number of major surfaces. Each major surface 14, 16 can take any suitable shape or shapes and have any suitable dimensions. In one or more embodiments, at least one of the first or second major surfaces 14, 16 can be planar. In one or more embodiments, one or more portions of at least one of the first or second major surfaces 14, 16 can be curved.

Extending between the first major surface 14 and the second major surface 16 of the housing 12 is the sidewall 18. The sidewall 18 can take any suitable shape or shapes and have any suitable dimensions. The housing 12 can have any suitable number of sidewalls, e.g., one, two, three, four, five or more sidewalls.

Disposed in the sidewall 18 are the one or more openings 20. The openings 20 can be disposed in one or more portions of the sidewall 18. For example, as shown in FIG. 1, openings 20 are disposed in each side portion of the sidewall 18. Any suitable number of openings 20 can be disposed in the sidewall 18. Further, each opening 20 can take any suitable shape or shapes and have any suitable dimensions. In one or more embodiments, each opening 20 has the same shape and dimensions. In one or more embodiments, one or more openings 20 has at least one of a shape or dimensions that are different from one or more additional openings. The openings 20 can be arranged in a pattern or an array. Although depicted as being disposed only in the sidewall 18, one or more openings 20 can be disposed in one or more of the major surfaces 14, 16.

The openings 20 can be disposed in the sidewall 18 using any suitable techniques, e.g., drilling, ablation, laser ablation, hole punching, machining, etc. The openings 20 can also be assembled as sub-components by bonding nonconductive material to a metal ring that is bonded to the housing 12 using, e.g., laser welding. In one or more embodiments, the housing 12 can be formed such that the sidewall 18 is molded to include one or more openings 20 using any suitable molding technique, e.g., injection molding, metal injection molding, laser assisted additive manufacturing, etc.

One or more openings 20 can be hermetically sealed by the nonconductive material 24 that is disposed on the housing 12. In one or more embodiments, each opening 20 is hermetically sealed by the nonconductive material 24. Any suitable technique or techniques can be utilized to hermetically seal one or more openings 20 with the nonconductive material 24, e.g., diffusion bonding, laser-assisted diffusion bonding, adhering, mechanically fastening, brazing, glassing, etc.

For example, the nonconductive material 24 can be hermetically sealed to the housing 12 using one or more of the diffusion bonding techniques described in co-owned and co-filed U.S. Patent Application No. 10,124,559 to Sandlin et al. and entitled KINETICALLY LIMITED NANO-SCALE DIFFUSION BOND STRUCTURES AND METHODS. In one or more embodiments, electromagnetic radiation (e.g., light) can be directed through the nonconductive material 24 and focused on a region between the nonconductive material and housing 12. Any suitable electromagnetic radiation can be utilized to form the bond. In one or more embodiments, the electromagnetic radiation can include laser light that can include any suitable wavelength or range of wavelengths. In one or more embodiments, the laser light can include light having a wavelength of at least 200 nm. In one or more embodiments, the laser light can include a wavelength of no greater than 2000 nm. For example, laser light can include UV light, visible light, IR light, and combinations thereof. The UV light can be provided by a UV laser that has any suitable wavelength or range of wavelengths and any suitable pulse width. In one or more embodiments, a UV laser can be utilized to provide light having a wavelength in a range of 100-400 nm and a pulse width in a range of 1-100 ns. In one or more embodiments, the materials for the housing 12 and the nonconductive material 24, and the power level and wavelength of the light used may be selected such that the light may not directly damage, ablate, warp, or cut the substrate and the housing, and such that the substrate and the housing retain their bulk properties.

In general, electromagnetic radiation can be provided by any suitable laser or laser system. For example, the laser may generate electromagnetic radiation having a relatively narrow set of wavelengths (e.g., a single wavelength). In one or more embodiments, the electromagnetic radiation emitted by the laser may form a collimated beam that may not be focused on a particular point. In one or more embodiments, the electromagnetic radiation emitted by the laser may be focused on a focal point at a region between nonconductive material 24 and the housing 12 to generate a laser bond.

Although the laser may provide electromagnetic radiation that has a narrow range of wavelengths, in one or more embodiments, the laser may represent one or more devices that emit electromagnetic radiation having a wider range of wavelengths than a single typical laser. A wide variety of devices may be used to emit electromagnetic radiation having a narrow or wide range of wavelengths. In one or more embodiments, the laser may include one or more laser devices including diode and fiber lasers. Laser sources may also include, e.g., Ti sapphire lasers, argon ion lasers, Nd:YAG lasers, XeF lasers, HeNe lasers, Dye lasers, GaAs/AlGaAs lasers, Alexandrite lasers, InGaAs lasers, InGaAsP lasers, Nd:glass lasers, Yb:YAG lasers, and Yb fiber lasers. The laser device may also include one of continuous wave, modulated, or pulsed modes. Accordingly, a wide variety of laser devices may be used in the bonding process. In one or more embodiments, a power level of the laser may be set to approximately 1 W, distributed across the approximate focused beam diameter of 10 µm, with a top hat, Gaussian, or other suitable spatial energy profile.

The nonconductive material 24 can include any suitable material or materials, e.g., at least one of sapphire, glass, zirconia, or alumina, or other nonconductive, biocompatible, biostable material. Further, nonconductive material 24 can be disposed on the housing 12 using any suitable technique or techniques. In one or more embodiments, the nonconductive material 24 can be formed separately from the housing 12 and then disposed on the housing using any suitable technique or techniques.

In one or more embodiments, one or more of the openings 20 can include a feedthrough (not shown). Such feedthrough can provide any electrical connection between an outer surface of the housing 12 and an interior of the housing. Any suitable technique or techniques can be utilized to form a feedthrough in one or more of the openings 20. Further, any suitable electronic component or cable can be electrically connected to one or more charging coils or electrical components disposed within the housing 12 utilizing the feedthrough.

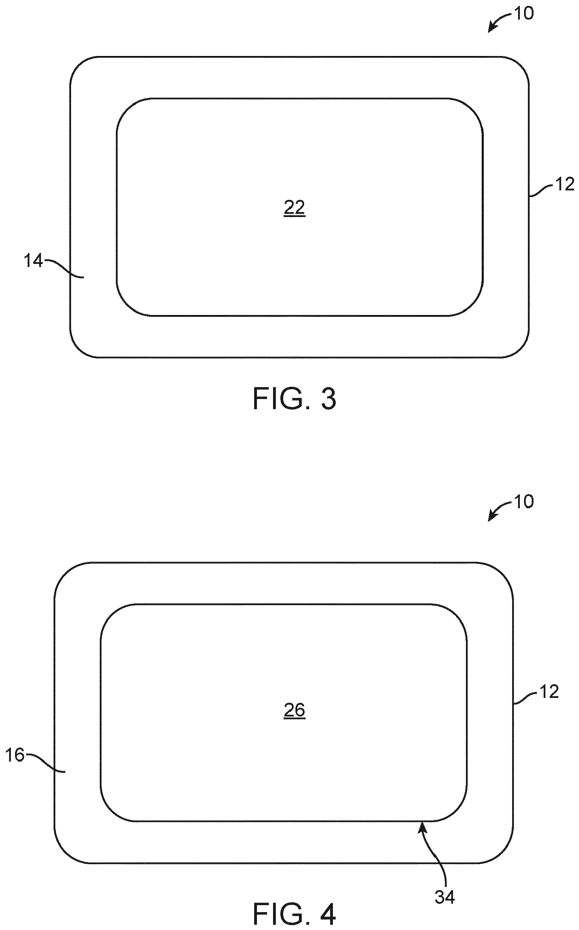

Also disposed on the housing 12 is the window 22. Such window 22 can be disposed on any suitable portion or portions of the housing 12. In one or more embodiments, the window 22 can be disposed in or over a window opening 32 FIG. 6) disposed in the housing 12. The window opening 32 can be disposed in the housing 12 using any suitable technique or techniques, e.g., the same techniques described herein regarding the openings 20. In one or more embodiments, the window 22 can be disposed on at least one of the first major surface 14 or the second major surface of the housing 16. As shown in FIG. 4, the window 22 is disposed on the first major surface 14 of the housing 12, and a second window 26 can be disposed on the second major surface 16 of the housing as shown in FIG. 5. Any suitable number of windows can be disposed on the housing 12.

The window 22 can take any suitable shape or shapes and have any suitable dimensions. Further, the window 22 can be disposed on the housing 12 such that an outer surface 23 of the window is in a plane defined by the first major surface 14 of the housing 12, i.e., the outer surface of the window is flush with the first major surface of the housing. In other words, the window 22 can be disposed on the first major surface 14 of the housing 12 such that it is recessed within the first major surface. This can be seen, e.g., in FIG. 6, where the window 22 is disposed on the first major surface 14 of the housing 12 such that the outer surface 23 of the window is in the same plane as the first major surface of the housing. Any suitable technique or techniques can be utilized such that the window 22 is disposed on the first major surface 14 such that it is recessed within the first major surface. For example, as shown in FIG. 6, the housing 12 includes a recessed surface 28 spaced apart from the first major surface 14 that is adapted to receive the window 22. The window 22 can be in contact with the recessed surface 28. In one or more embodiments, the window 22 can be connected to the recessed surface 28 as is further described herein.

The window 22 can include any suitable material or materials. In one or more embodiments, the window 22 can include a nonconductive material, e.g., the same materials described herein regarding the nonconductive material 24 such as at least one of sapphire, glass, zirconia, alumina, etc. Further, the window 22 can be a monolithic layer that can be formed using any suitable technique or techniques. In one or more embodiments, the window 22 can include two or more layers that are connected together using any suitable technique or techniques.

Further, the window 22 can be hermetically sealed to the housing 12 using any suitable technique or techniques, e.g., the same techniques described herein regarding hermetically sealing the nonconductive material 24 to the housing. For example, in one or more embodiments, the window 22 can be hermetically sealed to the housing 12 using a laser assisted diffusion bonding process such that the window is diffusion bonded to the housing. In one or more embodiments, the window 22 is laser assisted diffusion bonded to the housing 12 such that a bond line 30 is formed between the window and the housing as is shown in FIG. 6. The bond line 30 can take any suitable shape or shape and have any suitable dimensions. Further, in one or more embodiments, the window 22 can be brazed to the housing 12 using any suitable technique or techniques.

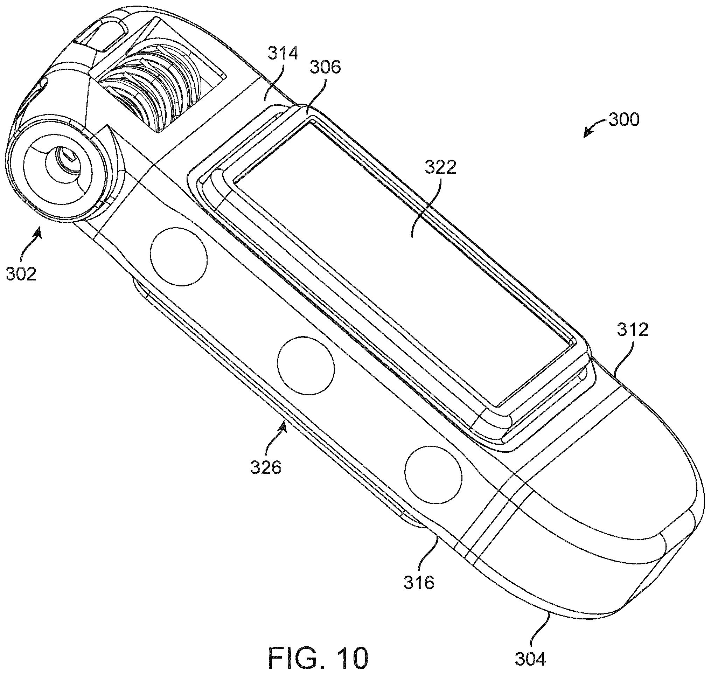

As mentioned herein, the device 10 can include any suitable number of windows. For example, in the embodiment illustrated in FIGS. 1-6, the device 10 includes the window 22 and the second window 26. The second window 26 can include any suitable window, e.g., window 22. All of the design considerations and possibilities described herein regarding the window 22 apply equally to the second window 26. The second window 26 can have the same shape or shapes as the window 22 or different shapes. Further, the second window 26 can have the same dimensions as window 22 or different dimensions. In one or more embodiments, the second window 26 can be disposed in or over a second window opening 34 disposed in the housing 12. Such window opening 34 can be disposed in the housing 12 using any suitable technique or techniques. In one or more embodiments, at least one of the first window 22 or second window 26 can be manufactured as a separate component by bonding the window to a metal frame (e.g., frame 306 of FIG. 10) using any suitable technique, e.g., laser diffusion bonding, thermal diffusion bonding, glassing, etc. The window and frame can be bonded to the housing 12 using any suitable technique, e.g., the same techniques utilized to bond the window to the metal frame.

At least one of the window 22, the second window 26, or one or more of the sealed openings 20 can be adapted to transmit electromagnetic energy in any suitable portion or portions of the electromagnetic radiation spectrum. In one or more embodiments, at least one of the windows 22, 26 or one or more of the sealed openings 20 can be adapted to transmit radio-frequency energy.

The device 10 can include any suitable component or components disposed on or within the housing 12. For example, the device 10 can include a charging coil 36 disposed on or within the housing 12. As shown in FIG. 5, which is a cross-section plan view of the device 10, the charging coil 36 is disposed within the housing 12. In one or more embodiments, the charging coil 36 can be disposed on one or both of the window 22 or the second window 26 or on or within one or more of the openings 20. The charging coil 36 can include any suitable charging coil or coils. The coil 36 can be utilized to inductively couple the device 10 with an external inductive charging system for charging the device when it is implanted within the body of the patient or for telemetry or other types of communication with a transceiver that is external to the patient's body. The charging coil 36 is adapted to receive electromagnetic energy directed through at least one of the windows 22, 26 or one or more of the sealed openings 20.

In one or more embodiments, the device 10 can also include a second or additional charging coils 37 (FIG. 2). The second charging coil 37 can be disposed in any suitable location on or within the housing 12. In the embodiment illustrated in FIG. 2, the second charging coil 37 is disposed on external or internal surfaces of the nonconductive material 24 disposed within the opening 20 or within the nonconductive material. The device 10 can include any suitable number of charging coils 36, 37. For example, additional openings 20 can include a charging coil to provide charging capabilities in more than one direction relative to the orientation of the housing 12. Such additional charging coils can provide charging of the device 10 regardless of device orientation as is further described, e.g., in U.S. Patent No. 10,821,292 to Iyer et al. and entitled MULTI-AXIS COIL FOR IMPLANTABLE MEDICAL DEVICE.

The device 10 can also include one or more electronic components 38 (FIG. 5) disposed on or within the housing 12. The electronic component 38 can be electrically connected to the charging coil 36. The electronic component 38 can include any suitable electronic device or component, e.g., sensing circuitry for sensing electrical activity via one or more electrodes and therapy generation circuitry for delivering electrical stimulation therapy via the electrodes. Electronic component 38 can include any discrete and/or integrated electronic circuit components that implement analog and/or digital circuits capable of producing the functions attributed to the device 10 described herein. In one or more embodiments, housing 14 can also house components for sensing other physiological parameters, such as acceleration, pressure, sound, and/or impedance. In one or more embodiments, the electronic component 38 can include one or more capacitors, resistors, diodes, integrated circuits, controllers, processors, sensors (e.g., temperature sensor), batteries, etc.

The electronic component 38 can be electrically connected to the charging coil 36 using any suitable technique or techniques. In general, current induced in the charging coil 36 by an electromagnetic field applied by an external component of an energy transfer system can charge the electronic component 38, thereby storing energy within the device 10 or elsewhere within the body. Such energy can be utilized to provide power to additional electronic components disposed on or within the housing 12 or connected to the housing.

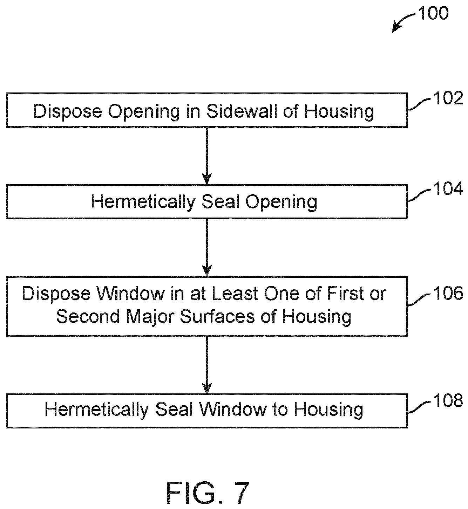

Any suitable technique or techniques can be utilized to form the implantable medical device 10. For example, FIG. 7 is a flowchart of one method 100 of forming the implantable medical device 10 of FIGS. 1-6. Although described in regard to the implantable medical device 10, the method 100 can be utilized to form any suitable implantable medical device. At 102, the one or more openings 20 can be disposed in the sidewall 18 of the housing 12 using any suitable technique or techniques. One or more of the openings 20 can be hermetically sealed with the nonconductive material 24 using any suitable technique or techniques at 104. Further, the window 22 can be disposed on or in the first major surface 14 of the housing 12 using any suitable technique or techniques at 106. Prior to or after disposing the window 22 on or in the first major surface 14 of the housing 12, the window opening 32 can be disposed in the housing using any suitable techniques, and the window can be disposed over or within the window opening. In one or more embodiments, the second window 26 can be disposed on or in the second major surface 16 of the housing 12 using any suitable techniques. Further, the window opening 34 can be disposed in the housing 12 using any suitable technique or techniques, and the second window 26 can be disposed over or within the window opening. In one or more embodiments, the window 22 and the second window 26 can be hermetically sealed to the housing 12 using any suitable techniques at 108.

Although not shown in FIG. 7, the method 100 can further include disposing the charging coil 36 on or within the housing 12 using any suitable technique or techniques. In one or more embodiments, the charging coil 36 is disposed within the housing 12 prior to hermetically sealing one or both of the windows 22, 26. Further, one or more electronic components 38 can be disposed within the housing 12 using any suitable technique or techniques prior to one or both of the windows 22, 26 being hermetically sealed to the housing. In one or more embodiments, the housing 12 can include two or more portions or parts that are connected together using any suitable technique or techniques. In such embodiments, at least one of the charging coil 36 or electronic component 38 can be disposed within one of these portions of the housing, and one or more additional portions can be connected together to form the housing such that the charging coil and electronic component are disposed within the housing. Further, the electronic component 38 can be electrically connected to the charging coil 36 using any suitable technique or techniques.

In one or more embodiments, the method 100 can also include directing electromagnetic energy through at least one of the window 22, the second window 26, or one or more of the openings 20 of the housing 12 to the charging coil 36 using any suitable technique or techniques.

As mentioned herein, one or more external components such as leads or electrodes can be connected to electronic components disposed within a housing of an implantable medical device using any suitable technique or techniques. For example, FIG. 10 is a schematic perspective view of another embodiment of an implantable medical device 300. All of the design considerations and possibilities described herein regarding the implantable medical device 10 of FIGS. 1-6 apply equally to the implantable medical device 300 of FIG. 10. One difference between implantable medical device 300 and implantable medical device 10 is that housing 312 includes a lead port 302 that is adapted to be connected to a lead or cable. The lead port 302 is further adapted to electrically connect a lead inserted into the port with one or more electronic components disposed within the housing 312. Another difference between implantable medical device 300 and device 10 is that the housing 312 also includes an external battery 304 that is connected to the housing 312 using any suitable technique or techniques. The external battery 304 can be electrically connected to one or more electronic components disposed within the housing 312.

Further, the device 300 includes a window 322 that is connected to a frame 306. The window 322 can be connected to the frame 306 using any suitable technique or techniques. Further, the frame 306 can include any suitable material or materials, e.g., at least one of a metallic, polymeric, or inorganic material. The frame 306 can be connected to a first major surface 314 of the housing 312 using any suitable technique or techniques. In one or more embodiments, a second window 326 can be disposed on a second major surface 316 of the housing 312 using a frame similar to the frame 306 that connects the window 322 to the first major surface 314 of the housing.

The various embodiments of implantable medical devices described herein can be utilized with any suitable system. For example, FIGS. 8-9 are schematic views of one embodiment of a wireless energy transfer system 200. The system 200 can include any suitable wireless energy transfer system, e.g., one or more of the systems described in U.S. Patent No. 10,143,788 B2, entitled TRANSCUTANEOUS ENERGY TRANSFER SYSTEMS.

The system 200 includes the implantable medical device 10 of FIGS. 1-6 and external components 240. In FIG. 8, the external components 240 of the system 200 are illustrated, and in FIG. 9, the implantable medical device 10 of the system is illustrated as being implanted within a body 202 of a patient 204. The external components 240 can include an external module 242 and a primary coil 244. In one or more embodiments, the primary coil 244 can be disposed in a separate housing 246 from the external module 242. The external module 242 can be located in any suitable location relative to the patient's body 202, e.g., around the patient's hip (e.g., in a pocket of the patient's clothing, mounted to a belt of the patient, etc.), and the primary coil 244 can be located in any suitable location relative to the patient's body 202, e.g., on the patient's chest and secured in place by a garment worn by the patient, such as a sling or vest. The external module 242 and primary coil 244 are further connected to each other by a wire 248. Also shown in FIG. 8 is a clinical monitor 250, which can be worn, e.g., on the patient's wrist. In other examples, the clinical monitor 250 can be located elsewhere, such as in the external module, or in the patient's smartphone, or not on the patient altogether.

In the embodiment illustrated in FIG. 8, an external battery and external electronics (not shown) can be disposed in a housing 252 of the external module 242. In one or more embodiments, the external battery may be disposed in a separate housing (e.g., separately mounted to the outside of the patient) and wired to the external module 242.

Although the system 200 includes the implantable medical device 10 of FIGS. 1-6, the system can include any suitable electronic device or package described herein. As illustrated in FIG. 9, the implantable medical device 10 can include the charging coil 36 (i.e., secondary coil) disposed within the housing 12, a pump 254, and an electronic module 256 electrically connected to the housing and the pump. In one or more embodiments, each of the housing 12, the pump 254, and the electronic module 256 can be disposed in a separate housing and dispersed throughout the patient's body 202 to accommodate the anatomy of the patient. For example, in the embodiment illustrated in FIG. 9, the housing 12 is mounted in the patient's chest. In one or more embodiments, the housing 12 can be mounted to the patient's rib, back, abdomen, or muscle in any subcutaneous plane. Although depicted as being disposed in separate locations, the pump 254 and the electronic module 256 can be disposed within the housing 12 of the implantable medical device 10.

The housing 12 is electrically connected to the electronic module 256 by a cable 258, and the pump 254 is electrically connected to the electronics module 256 by a second cable 260. The pump 254 can be connected, e.g., to a heart of the patient. Although not shown, the implantable medical device 10 can also include an implanted battery disposed in any suitable location within the patient's body 202 or within the housing 12. In one or more embodiments, the implanted battery is disposed within a housing 262 of the electronics module 256. In one or more embodiments, the implanted battery may be separately housed, and an additional wire may connect the electronics module 256 to the implanted battery.

The charging coil 36 is disposed within the housing 12 of the implantable medical device 10 and is adapted to be electromagnetically coupled to the primary coil 244. For example, the charging coil 36 can be adapted to be inductively coupled to the primary coil 244. Positioning of the charging coil 36 within the patient 204 can be done in such a manner that makes mounting the primary coil 244 in proximity to the secondary coil easy for the patient. For instance, the charging coil 36 can be positioned close to the skin of the patient 204. Moreover, the charging coil 36 can be positioned close to a relatively flat part of the patient's body 202 to make mounting the primary coil 244 easier. In the embodiment illustrated in FIG. 9, the charging coil 36 disposed within the housing 12 is positioned close to the front of the patient's chest such that mounting the primary coil 244 to the patient's chest places the primary coil proximate the secondary coil. In those examples where the housing 12 is mounted to the patient's rib, back, or abdomen, the charging coil 36 can be external to the housing and connected to the electronic component 38 disposed within the housing by a cable that is electrically connected to a feedthrough disposed in the housing. In such embodiments, the charging coil 36 can be located close to the patient's skin, such that the primary coil 244 can be mounted in close proximity.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include computer-readable storage media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. An implantable medical device comprising:
a housing comprising a first major surface and a second major surface, a sidewall that extends between the first major surface and the second major surface, and an opening disposed in the sidewall;
a window disposed on at least one of the first major surface or the second major surface of the housing; and
a nonconductive material disposed on the housing, wherein the opening is hermetically sealed by the nonconductive material;
wherein at least one of the window or the sealed opening is adapted to transmit electromagnetic energy.

2. The device of claim 1, further comprising a charging coil disposed within the housing and adapted to receive electromagnetic energy directed through the window or the sealed opening.

3. The device of claim 2, further comprising an electronic component disposed within the housing and electrically connected to the charging coil.

4. The device of any one of claims 1 to 3, wherein the window comprises at least one of sapphire, glass, zirconia, or alumina.

5. The device of any one of claims 1 to 4, wherein the housing comprises titanium.

6. The device of any one of claims 1 to 5, wherein the housing comprises a plurality of openings disposed in the sidewall, wherein each opening is hermetically sealed by the nonconductive material.

7. A pacemaker comprising the implantable medical device of any one of claims 1 to 6.

8. A wireless energy transfer system comprising:
an external charging component adapted to emit electromagnetic energy; and
an implantable medical device comprising:
a housing comprising a first major surface and a second major surface, a sidewall that extends between the first major surface and the second major surface, and an opening disposed in the sidewall;
a window disposed on at least one of the first major surface or second major surface of the housing;
nonconductive material disposed on the housing, wherein the opening is hermetically sealed by the nonconductive material;
a charging coil disposed within the housing and adapted to receive the electromagnetic energy emitted by the external charging component; and
an electronic component disposed within the housing and electrically connected to the charging coil;
wherein at least one of the window or the sealed opening is adapted to transmit electromagnetic energy to the charging coil disposed within the housing.

9. The system of claim 8, wherein the window of the implantable medical device is disposed in the first major surface of the housing of the device, and further wherein the housing comprises a second window disposed in the second major surface of the housing.

10. The system of claim 8 or 9, wherein the housing of the implantable medical device comprises a plurality of openings disposed in the sidewall, wherein each opening is hermetically sealed by the nonconductive material.

11. The system of any one of claims 8 to 10, wherein the window of the implantable medical device comprises at least one of sapphire, glass, zirconia, or alumina.

12. The system of any one of claims 8 to 11, wherein the nonconductive material comprises at least one of sapphire, glass, zirconia, or alumina.

13. The system of any one of claims 8 to 12, wherein the window is hermetically sealed to the housing.

14. The system of any one of claims 8 to 13, wherein the implantable medical device comprises a leadless cardiac monitor.
